# EUROPEAN PATENT SPECIFICATION

(11) **EP 3 937 787 B1**
(45) Date of publication and mention of the grant of the patent: **29.05.2024**
(21) Application number: 20770721.7
(22) Date of filing: 13.03.2020
(51) Int. Cl.: A61B 8/00, A61B 8/14, A61B 90/50, A61B 8/08, A61B 90/00

(54) **PIVOT GUIDE FOR ULTRASOUND TRANSDUCER**
SCHWENKFÜHRUNG FÜR ULTRASCHALLWANDLER
GUIDE DE PIVOT POUR TRANSDUCTEUR ULTRASONORE

(30) Priority: 14.03.2019 US 201962818599 P
(43) Date of publication of application: 19.01.2022
(73) Proprietor: Sonic Incytes Medical Corp., Vancouver BC V5Z 1M9 (CA)
(72) Inventor: HONARVAR, Mohammad, Vancouver BC V5Z 1M9 (CA); SCHNEIDER, Caitlin Marie, Vancouver BC V5Z 1M9 (CA); LOBO, Julio Raul, Vancouver BC V5Z 1M9 (CA)
(74) Representative: Eisenführ Speiser
(86) International application number: PCT/CA2020/050346
(87) International publication number: WO 2020/181394

(56) References cited:
- WO-A1-2004/006773
- US-A1- 2006 020 211

## Description

### Field

This disclosure relates to apparatus and methods for positioning and/or tracking an ultrasound transducer relative to a patient. The invention is defined in claim 1, with further aspects in the dependent claims.

### Background

Ultrasound imaging is often used for quick diagnostic imaging of a patient. It is often desirable to acquire a three dimensional volume of ultrasound imaging data for diagnostic purposes. For example, the acquired three dimensional ultrasound imaging data may comprise sets of: B-Mode images, color Doppler images, displacement data caused by shear waves used during elastography measurements, etc.

The creation of three dimensional volumes of ultrasound imaging data may increase a field of view that is captured. Increasing the field of view may allow for more complex three dimensional tissue relationships to be visualized and measured. During elastography imaging, acquiring three dimensional ultrasound imaging data may, for example, allow for shear wave propagation tracking over a volume represented by the three dimensional ultrasound imaging data.

Some systems for tracking movement of an ultrasound transducer in three dimensions are known. For example, a six degree-of-freedom tracking system such as a passive arm-linkage, an optical tracker, etc. may be used. Such systems are expensive and complex.

There remains a need for improved and less costly apparatus and methods for positioning and/or tracking an ultrasound transducer relative to a patient.

US200602021 1A1 discloses a base unit including a guide mechanism for guiding a puncture needle and a moving mechanism for movably supporting a holder to move a wave transmitting and receiving region of an ultrasonic wave relative to a puncture route of the puncture needle.

### Summary

The present disclosure has a number of aspects. These include, without limitation:
- Guides useful for orienting and/or supporting an ultrasound transducer to obtain ultrasound images of a patient, particularly 3D ultrasound images or ultrasound image data that can be processed to provide 3D ultrasound image data sets or ultrasound images obtained by imaging through an intercostal space of a patient.
- Ultrasound transducer assemblies useful for obtaining ultrasound images of a patient, particularly 3D ultrasound images or ultrasound image data that can be processed to provide 3D ultrasound image data sets or ultrasound images obtained by imaging through an intercostal space of a patient.
- Methods for obtaining ultrasound images of a patient, particularly 3D ultrasound images or ultrasound image data that can be processed to provide 3D ultrasound image data sets or ultrasound images obtained by imaging through an intercostal space of a patient.

One aspect of the technology described herein provides an ultrasound transducer assembly. The ultrasound transducer assembly may comprise first and second protrusions respectively located at first and second ends of a transducer array of the ultrasound transducer on a pivot axis. The first and second protrusions may each comprise a patient-contacting surface that projects forwardly relative to the front surface of the transducer array. The first and second protrusions may be operable to indent skin of a patient adjacent to a region of the patient to be imaged by the ultrasound transducer when the ultrasound transducer is pressed against the skin of the patient.

In some embodiments the pivot axis is aligned with a front surface of the transducer array.

In some embodiments the pivot axis lies in an imaging plane of the ultrasound transducer.

In some embodiments the first and second protrusions are parts of a guide that is removably attached to the ultrasound transducer.

In some embodiments the guide comprises a body defining a cavity shaped to receive an end of the ultrasound transducer that includes the transducer array. The cavity may have an opening aligned with the transducer array when the transducer is inserted into the cavity. The first and second protrusions are respectively mounted at first and second opposing ends of the body.

In some embodiments the cavity is formed to provide a positive stop when the front face of the transducer array is aligned with the pivot axis.

In some embodiments the body comprises a plurality of resilient fingers spaced around an opening of the cavity, the resilient fingers dimensioned to flex when the end of the transducer is inserted into the cavity.

In some embodiments a patient-contacting surface of each of the protrusions has a radius of curvature substantially equal to a distance by which the patient-contacting surface projects forwardly relative to the front surface of the transducer array.

In some embodiments the radius of curvature is in the range of about 0.5 cm to 1 cm.

In some embodiments the patient-contacting surfaces of the first and second protrusions each comprises a cylindrical configuration.

In some embodiments the first and second protrusions are each mounted to pivot relative to the transducer.

In some embodiments each of the first and second protrusions comprises a roller mounted to rotate about the pivot axis.

In some embodiments the ultrasound transducer comprises a handle pivotally mounted to the transducer.

In some embodiments the handle extends generally perpendicularly to the pivot axis.

In some embodiments the handle is pivotal relative to the body about the pivot axis.

In some embodiments the handle comprises first and second arms that are respectively pivotally mounted to the transducer adjacent to the first and second ends of the transducer array.

In some embodiments the protrusions are provided by end portions of the first and second arms.

In some embodiments the handle comprises a frame and the frame includes one or more stops positioned to limit angular travel of the transducer relative to the frame.

In some embodiments the first and second protrusions are detachably mounted to the transducer.

In some embodiments the first and second protrusions are integral with a case of the ultrasound transducer.

In some embodiments the ultrasound transducer comprises an angle sensor connected to measure an angle of inclination of the transducer.

In some embodiments wherein the angle sensor comprises an IMU.

Another aspect of the technology described herein provides an ultrasound transducer assembly comprising a transducer array that is elongated in a first direction and a first patient-contacting member attached to the transducer at one end of the transducer array. The transducer array and first patient-contacting member may be operable to indent skin of a patient adjacent to a region of the patient to be imaged by the ultrasound transducer when the ultrasound transducer is pressed against the skin of the patient thereby permitting pivoting of the ultrasound transducer about a pivot axis parallel to the first direction while resisting rotation of the transducer assembly about an axis that is perpendicular to the first direction.

In some embodiments the transducer array and first patient-contacting member are dimensioned to engage between ribs of a patient.

In some embodiments the transducer array and first patient-contacting member are spaced apart from one another.

In some embodiments the pivot axis is aligned with a front surface of the transducer array.

In some embodiments the pivot axis lies in an imaging plane of the ultrasound transducer.

In some embodiments the first patient-contacting surface is part of a guide that is removably attached to an ultrasound transducer of which the ultrasound transducer array is a part.

In some embodiments the guide comprises a body defining a cavity shaped to receive an end of the ultrasound transducer that includes the transducer array. The cavity may have an opening aligned with the transducer array when the transducer is inserted into the cavity. The first patient-contacting surface may be mounted at a first end of the body.

In some embodiments a surface of the ultrasound transducer array and the patient-contacting surface each comprise generally cylindrical surfaces having equal radii of curvature.

In some embodiments the radius of curvature is in the range of about 0.5 cm to 2 cm.

In some embodiments the patient-contacting surface is a surface of a member that is mounted to pivot relative to the transducer array.

In some embodiments the patient-contacting surface comprises a surface of a roller mounted to rotate about the pivot axis.

Another aspect of the technology described herein provides a guide for guiding pivotal movement of an ultrasound transducer relative to a volume to be imaged. The guide may comprise a body configured to couple to an end of the ultrasound transducer that includes a transducer array and first and second protrusions respectively located at first and second ends of the body. The first and second protrusions may be located at first and second ends of the transducer array. A pivot axis may be defined by the first and second protrusions such that when the body is coupled to the ultrasound transducer the ultrasound transducer is pivotable relative to the pivot axis. Each of the first and second protrusions may comprise a patient-contacting surface that projects forwardly relative to the pivot axis. The first and second protrusions may be operable to indent skin of a patient adjacent to a region of the patient to be imaged by the ultrasound transducer when the ultrasound transducer is pressed against the skin of the patient.

In some embodiments the pivot axis is aligned with a front surface of the transducer array.

In some embodiments the pivot axis is in an imaging plane of the transducer array.

In some embodiments the body is removably attachable and detachable from the ultrasound transducer.

In some embodiments the body defines a cavity shaped to receive the end of the ultrasound transducer that includes the transducer array. The cavity may have an opening aligned with the transducer array when the transducer is inserted into the cavity.

In some embodiments the cavity is formed to provide a positive stop when the front face of the transducer array has a desired alignment with the pivot axis.

In some embodiments the body comprises a plurality of resilient fingers spaced around an opening of the cavity. The resilient fingers may be dimensioned to flex when the end of the transducer is inserted into the cavity.

In some embodiments a patient-contacting surface of each of the protrusions has a radius of curvature substantially equal to a distance by which the patient-contacting surface projects forwardly relative to the front surface of the transducer array.

In some embodiments the radius of curvature is in the range of about 0.5 cm to 1 cm.

In some embodiments the patient-contacting surfaces of the first and second protrusions each comprises a cylindrical configuration.

In some embodiments the first and second protrusions are each mounted to pivot relative to the body.

In some embodiments each of the first and second protrusions comprises a roller mounted to rotate about the pivot axis.

In some embodiments the guide comprises a handle pivotally mounted to the body.

In some embodiments the handle extends generally perpendicularly to the pivot axis.

In some embodiments the handle is pivotal relative to the body about the pivot axis.

In some embodiments the handle comprises first and second arms that are respectively pivotally mounted to the body adjacent to the first and second ends of the body.

In some embodiments the protrusions are provided by end portions of the first and second arms.

In some embodiments the handle comprises a frame and the frame includes one or more stops positioned to limit angular travel of the body relative to the frame.

In some embodiments the first and second protrusions are detachably mounted to the body.

In some embodiments the guide comprises a mechanism for adjusting a distance by which at least one of the first second protrusions projects forwardly from the pivot axis.

In some embodiments the first and second protrusions have dimensions parallel to the pivot axis in the range of ½ cm to 2 cm.

Another aspect of the technology described herein provides a guide for guiding pivotal movement of an ultrasound transducer relative to a volume to be imaged. The guide may comprise a body configured to couple to an end of the ultrasound transducer that includes a transducer array and at least a first patient contacting surface coupled to one end of the body. The patient contacting surface may be dimensioned to be received between ribs of a patient to establish a reference point.

In some embodiments the guide comprises a hinge coupling the patient-contacting surface to the body. The hinge may be arranged to allow pivotal motion of the body relative to the patient contacting surface about an axis that is parallel to an axis of the transducer array when the transducer array is coupled to the end of the transducer.

In some embodiments the hinge comprises a snap hinge comprising first and second hinge elements that are detachably coupled together.

In some embodiments the body is dimensioned to engage an intercostal space of the patient.

In some embodiments the body is removably attachable and detachable from the ultrasound transducer.

In some embodiments the body defines a cavity shaped to receive the end of the ultrasound transducer that includes the transducer array. The cavity may have an opening aligned with the transducer array when the transducer is inserted into the cavity.

In some embodiments the cavity is formed to provide a positive stop when the front face of the transducer array has a desired alignment with the pivot axis.

In some embodiments the body comprises a plurality of resilient fingers spaced around an opening of the cavity. The resilient fingers dimensioned to flex when the end of the transducer is inserted into the cavity.

In some embodiments the patient contacting surface comprises a cylindrical configuration.

In some embodiments the patient contacting surface is mounted to pivot relative to the body.

In some embodiments the patient contacting surface comprises a surface of a roller mounted to rotate relative to the body.

In some embodiments the guide comprises an angle sensor connected to measure an angle of inclination of the body.

In some embodiments the angle sensor comprises an IMU.

Another aspect of the technology described herein comprises a method for obtaining a 3D ultrasound image of a volume. The method may comprise pressing against a body of a patient an ultrasound transducer comprising a transducer array and first and second protrusions respectively adjacent to first and second ends of the transducer array such that the first and second protrusions indent a surface of the body of the patient and thereby resist translation of the first and second protrusions relative to the body of the patient. The method may also comprise pivoting the transducer about a pivot axis defined by the first and second protrusions while operating the transducer array to obtain a plurality of images of the body of the patient. Each of the images may correspond to a corresponding plane which passes through the pivot axis.

In some embodiments the method comprises monitoring an output of a sensor which varies according to an inclination of the transducer while pivoting the transducer about the pivot axis to determine an angular measure corresponding to each of the plurality of planes and associating the angular measures with the corresponding planes.

In some embodiments the method comprises combining the plurality of images into a three-dimensional data structure using the angular measures.

In some embodiments pressing the ultrasound transducer against the body of the patient comprises placing the first and second protrusions to engage an intercostal space such that a longitudinal axis of the transducer array is aligned with the intercostal space.

In some embodiments the plurality of planes pass through the intercostal space.

In some embodiments the plurality of images include images of a liver in the body of the patient.

In some embodiments the plurality of images comprise B-mode ultrasound images.

In some embodiments the method comprises vibrating the body to generate shear waves in the body of the patient wherein the plurality of images comprise elastography images.

In some embodiments the plurality of images comprise Doppler ultrasound images.

Another aspect of the technology described herein provides a method for obtaining a 3D ultrasound image of a volume. The method may comprise engaging a patient contacting surface attached to an ultrasound transducer between ribs of a patient and thereby aligning the ultrasound transducer with an acoustic window between the ribs.

In some embodiments the method comprises pivoting the transducer about the patient contacting surface while operating the transducer array to obtain a plurality of images of a body of the patient, each of the images corresponding to a corresponding plane wherein the planes corresponding to the images share at least one fixed point.

In some embodiments the method comprises monitoring an output of a sensor which varies according to an inclination of the transducer while pivoting the transducer to determine an angular measure corresponding to each of the plurality of planes and associating the angular measures with the corresponding planes.

In some embodiments the method comprises combining the plurality of images into a three-dimensional data structure using the angular measures.

Further aspects and example embodiments are illustrated in the accompanying drawings and/or described in the following description.

### Brief Description of the Drawings

The accompanying drawings illustrate non-limiting example embodiments of the invention.
Figure 1 is a perspective view of an ultrasound transducer guide according to an example embodiment of the invention.
Figures 2A, 2B and 2C are front, perspective and side views respectively of an example ultrasound transducer coupled with the guide of Figure 1.
Figure 3A is a schematic illustration of an example ultrasound transducer and the guide of Figure 1 positioned to engage an intercostal space of a patient.
Figure 3B is a schematic illustration of an example three-dimensional volume of acquired ultrasound imaging data.
Figures 4A and 4B are front and side views respectively of an ultrasound transducer guide according to another example embodiment of the invention coupled with an example ultrasound transducer.
Figures 5A and 5B are side and perspective views respectively of an ultrasound transducer guide according to another example embodiment of the invention coupled with an example ultrasound transducer.
Figure 6A is a perspective view of an ultrasound transducer guide according to another example embodiment of the invention.
Figures 6B, 6C and 6D are front, perspective and side views respectively of an example ultrasound transducer coupled to the guide of Figure 6A.
Figure 6E is a schematic illustration of an example ultrasound transducer and the guide of Figure 6A positioned to engage an intercostal space of a patient.
Figure 7A is a perspective view of an example frame coupled to the guide of Figure 6A.
Figures 7B, 7C and 7D are front, perspective and side views respectively of an example ultrasound transducer coupled to the guide of Figure 6A and frame of Figure 7A.
Figure 7E is a schematic illustration of an example ultrasound transducer, the guide of Figure 6A and the frame of Figure 7A positioned to engage an intercostal space of a patient.

### Detailed Description

Throughout the following description, specific details are set forth in order to provide a more thorough understanding of the invention. Well known elements have not been shown or described in detail to avoid unnecessarily obscuring the invention. Accordingly, the specification and drawings are to be regarded in an illustrative, rather than a restrictive sense.

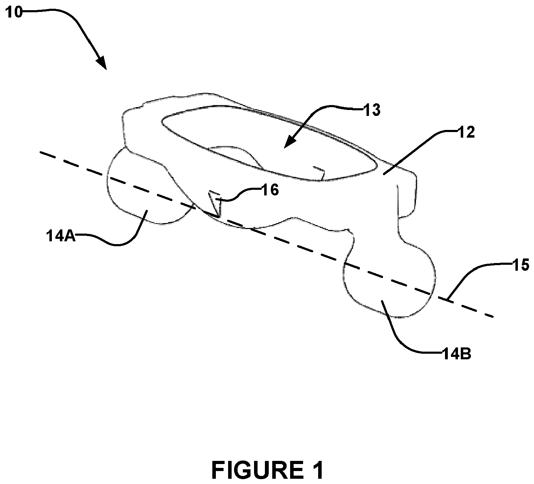

Figure 1 is a perspective view of an example ultrasound transducer guide 10. Body 12 of example guide 10 defines a cavity 13. Protrusions 14A and 14B (collectively or generally "protrusions 14") extend outwardly from opposing sides of body 12.

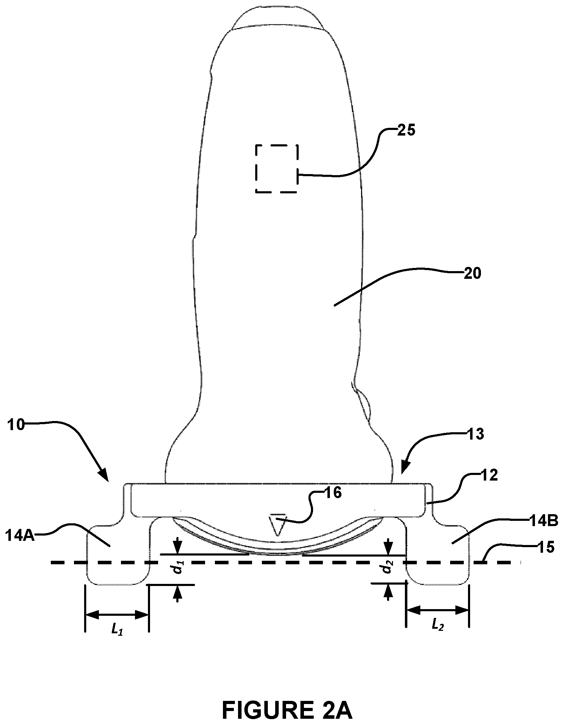

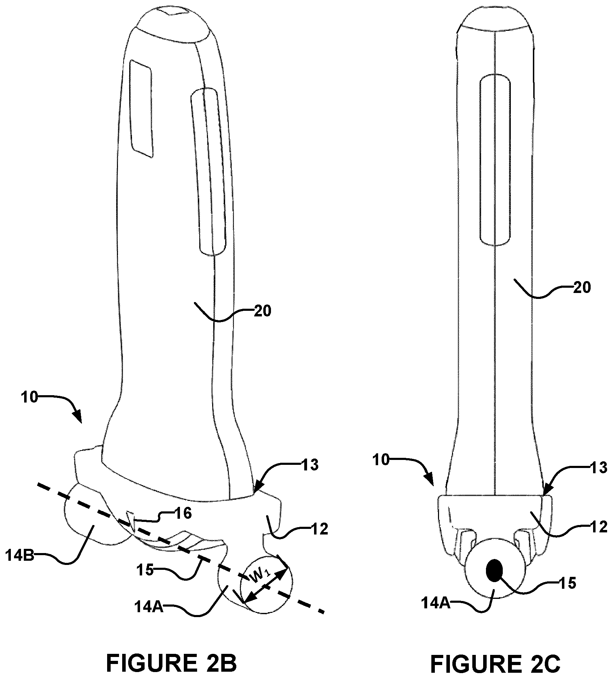

Cavity 13 is shaped to receive an end of an ultrasound transducer (e.g. an end of a transducer which comprises an imaging array). For example, end 20A of ultrasound transducer 20 may be received within cavity 13 as shown in Figures 2A-2C. Example transducer 20 is a curved transducer that may be used for abdominal imaging of a patient. However, cavity 13 and guide 10 may be shaped to receive other ultrasound transducers having different shapes.

The ultrasound transducer may have any suitable configuration. For example, the ultrasound transducer may be curved like ultrasound transducer 20. In some embodiments the ultrasound transducer is a straight linear transducer.

Protrusions 14 may project forward of a face of an ultrasound transducer. Additionally, or alternatively, protrusions 14 may be shaped to facilitate pivoting about an axis which is coincident with a face of an ultrasound transducer. Preferably, such axis is coincident with a transducer face. If the transducer face is not flat, such axis is preferably coincident with a forward most part of the transducer face. In some embodiments protrusions 14 have a curved lower surface (e.g. a cylindrical surface). In such embodiments a radius of curvature of the lower surface of protrusions 14 may be equal to an amount by which protrusions 14 project forward of the transducer face. In some embodiments a radius of curvature of the lower surface of protrusions 14 is in the range of about 0.5 cm to 1 cm. In some embodiments the radius of curvature is in the range of about 0.5 cm to 2 cm.

Once guide 10 is coupled to an ultrasound transducer (e.g. transducer 20), guide 10 and the transducer may be engaged with a body of a patient. Pressing guide 10 and/or the transducer against the body of the patient causes protrusions 14 to indent the patient's skin. This facilitates keeping guide 10 and the transducer at a desired position relative to the patient. Pressing guide 10 and/or the transducer against the body of the patient also positions a face of the transducer against the body of the patient such that the transducer can acquire ultrasound imaging data. A gel layer is typically applied between the patient's tissue and the ultrasound transducer.

Engagement of protrusions 14 with the patient's skin also defines an axis about which the transducer may be pivoted relative to the patient. This axis may be parallel to a longitudinal axis of an imaging array. The longitudinal axis of the imaging plane may be parallel to an imaging array. In some embodiments an axis extending through protrusions 14 (e.g. axis 15 shown in Figure 1) is parallel to the imaging plane.

In some embodiments pressing guide 10 against the body of the patient positions protrusions 14 and the face of the transducer to be generally parallel with a tissue region (e.g. an intercostal space) of the patient. "Generally parallel" means that an axis extending through protrusions 14 is offset from being parallel with the tissue region by no more than 15º. In some embodiments pressing guide 10 against the body of the patient positions protrusions 14 and the face of the transducer to be parallel with a tissue region (e.g. an intercostal space) of the patient.

In some embodiments pressing guide 10 against the body of the patient fixes and/or secures the axis of rotation of guide 10 and the axis of the imaging array relative to the patient during acquisition of ultrasound imaging data.

Typically guide 10 and/or the transducer are pressed hard enough for protrusions 14 to indent the outer skin layer of a patient sufficiently for the face of the transducer to be against the skin between protrusions 14. The transducer and the guide may then be pivoted about an axis extending through protrusions 14 (e.g. axis 15 shown in Figure 1). Such pivoting may, for example, facilitate obtaining an angular sweep of three dimensional ultrasound imaging data of a region of the patient.

For example, the acquired three dimensional ultrasound imaging data may comprise sets of: B-Mode images, color Doppler images, displacement data caused by shear waves used during elastography measurements, etc. In some cases guide 10 and the transducer are pressed against a body of a patient and pivoted relative to the patient while electrography measurements are being taken. Elastography may, for example, be performed as described in US patent publication No. 2014/0330122 to Baghani et al. entitled ELASTOGRAPHY USING ULTRASOUND IMAGING OF A THIN VOLUME and international PCT application published as WO2018/000103 to Salcudean et al. entitled ULTRASOUND SHEAR WAVE VIBRO-ELASTOGRAPHY OF THE ABDOMEN both of which are hereby cited.

Constraining pivoting of the transducer relative to an axis (e.g. axis 15) facilitates simple and/or accurate reconstruction of the acquired ultrasound imaging data. Typically guide 10 is designed to hold the transducer at an angle relative to axis 15 such that pivoting of the transducer is in an elevational (out-of-plane) direction relative to the acquired ultrasound imaging data.

Acquiring three-dimensional ultrasound imaging data typically also requires measuring a relative angle of an ultrasound transducer relative to an axis of rotation (e.g. axis 15). For example, inertial measurement units (IMUs) or other known angle measuring sensors (e.g. tilt sensors, gyroscopes, etc.) may be embedded inside an ultrasound transducer. The IMUs and/or measuring sensors may measure the relative angle of the ultrasound transducer as the transducer is pivoted to acquire the imaging data. As another example, acquired ultrasound image data may be processed to estimate changes in angle around axis 15.

The measured angles may be stored for reference during three dimensional reconstructions of the imaging data. However, since such sensors typically only accurately measure a relative angle of rotation, it is typically required to constrain the ultrasound transducer to pivot about a known axis (may be constrained about a fixed point as described elsewhere herein).

Example transducer 20 may optionally comprise one or more IMUs or measuring sensors 25 (see e.g. Fig. 2A). The one or more IMUs or measuring sensors 25 may be rigidly coupled to transducer 20. As transducer 20 is pivoted to collect ultrasound imaging data, IMU or measuring sensor 25 may measure a relative angle of transducer 20 between acquired planes of ultrasound imaging data. The measured angles may be associated with their corresponding planes of ultrasound imaging data during and/or after acquisition of the ultrasound imaging data. The measured angles and the ultrasound imaging data may be merged during three dimensional reconstruction of the ultrasound imaging data.

In some embodiments at least of the IMUs or measuring sensors 25 is coupled to guide 10. In some embodiments the IMUs or measuring sensors 25 may be rigidly coupled to transducer 20. In some embodiments an inclination sensor such as an IMU is attached to a guide that is attached to or attachable to transducer 20.

Preferably an axis extending centrally through protrusions 14 aligns with a face of the ultrasound transducer as described elsewhere herein. The face of the transducer may pivot about the axis extending through the protrusions. Having the transducer pivot about an axis that is fixed with respect to the face of the transducer typically simplifies three dimensional reconstruction of the acquired ultrasound imaging data (e.g. the forward most position of the ultrasound imaging data may be constant and aligned with the axis of rotation).

It is convenient but not necessary for the face of an ultrasound transducer to continuously contact the skin of a patient during pivoting of the transducer along an axis passing through the centre of the face. Maintaining the ultrasound transducer in continuous contact with the skin of the patient may reduce introduction of artefacts (e.g. additional artefacts caused by having additional transmission interfaces and/or boundaries).

As shown in Figure 2A, longitudinal centerlines of protrusions 14A and 14B are distances *d₁* and *d₂* respectively below an imaging face of transducer 20. Distances *d₁* and *d₂* may be the same or different. For example distances *d₁* and *d₂* may be different to account for different tissue densities (e.g. tissue surrounding one of protrusions 14A and 14B may compress more than tissue surrounding the other one of protrusions 14A and 14B). As another example distances *d₁* and *d₂* may be different to account for different elevations of the patient's body.

Distances *d₁* and *d₂* are large enough to facilitate sufficient indentation of protrusions 14 into a patient's tissue to hold guide 10 relative to the patient but small enough for the face of the ultrasound transducer to come into contact with the patient's tissue. Additionally, or alternatively, distances *d₁* and *d₂* are small enough for the face of the ultrasound transducer to come into contact with the patient's tissue without causing much discomfort for the patient.

In some embodiments protrusions 14 project by distances in the range of 0 and 2 cm below a face of an ultrasound transducer. In some embodiments protrusions 14 project by distances in the range of 0 and 1 cm below a face of an ultrasound transducer. In some embodiments protrusions 14 project by distances in the range of 0 and 0.5 cm below a face of an ultrasound transducer.

In some embodiments one or both of distances *d₁* and *d₂* is adjustable. For example, one or both of protrusions 14A and 14B may be coupled to body 12 of guide 10 using a mechanism having an adjustable length. In some embodiments the adjustable length mechanism comprises an indent mechanism. In some such embodiments engaging different indents varies how much a protrusion 14 extends below a face of the transducer. In some embodiments the adjustable length mechanism comprises a telescopic mechanism for varying distances *d₁* or *d₂.*

Protrusions 14A and 14B may also be defined by widths *w₁* and *w₂* respectively. Widths *w₁* and *w₂* may be the same or different. Protrusions 14A and 14B may each have uniform or non-uniform widths *w₁* and *w₂.* Widths *w₁* and *w₂* are typically equal to or smaller than a width of a face of the ultrasound transducer (e.g. so that the width of the ultrasound transducer limits pivoting rather than guide 10). Widths *w₁* and *w₂* may be large enough to prevent protrusions 14 from causing excessive patient discomfort when protrusions 14 are pressed against the patient's skin. In some embodiments widths *w₁* and *w₂* are in the range of about 0.5 to 2.5 cm. In some embodiments widths *w₁* and *w₂* are in the range of about 0.5 to 1 cm.

In some embodiments widths *w₁* and *w₂* of protrusions 14 are dimensioned to fit between two adjacent ribs of a patient. This facilitates aligning the ultrasound transducer with the space between the ribs. Aligning the transducer in such manner facilitates imaging through tissue between the ribs.

Protrusions 14A and 14B extend outwardly by lengths *L₁* and *L₂*. Lengths *L₁* and *L₂* may be the same or different. Lengths *L₁* and *L₂* are typically long enough to secure guide 10 relative to the patient but short enough to allow for positioning of guide 10 over a desired region of the patient's tissue (e.g. can still fit within the desired region). Additionally, or alternatively, lengths *L₁* and *L₂* are typically long enough to allow an operator to securely hold guide 10 in a desired position. In some embodiments lengths *L₁* and *L₂* are in the range of about 0.5 cm to 2.5 cm. In some embodiments lengths *L₁* and *L₂* are in the range of about 0.5 cm and 1 cm.

Increasing one or more of widths *w₁* and *w₂* and/or lengths *L₁* and *L₂* may improve stability of guide 10 while guide 10 is being pivoted relative to the patient.

In some embodiments an axis extending through protrusions 14 is in line with a face of a coupled ultrasound transducer. In some embodiments the axis extending through protrusions 14 is aligned with a longitudinal central axis of a face of a coupled ultrasound transducer.

Protrusions 14 are shown as being generally cylindrical in shape. However this is not necessary. In some embodiments only a bottom surface of protrusions 14 is curved (e.g. the surface which engages the patient's skin). In some such embodiments the top surface may have any shape (e.g. flat, curved, comprise ridges, etc.).

Making the bottom surface of protrusions 14 curved may increase patient comfort. However, this is not necessary. In some embodiments the bottom surface of protrusion 14 is not curved. In some such embodiments the bottom surface may be flat, wedge shaped, hexagonal, etc.

A range of angles through which guide 10 may be comfortably pivoted may be determined by a shape, width and/or curvature of the bottom surface of protrusions 14. In some embodiments guide 10 pivots between -30º and +30º. In some embodiments guide 10 pivots between -20º and +20º. In some embodiments guide 10 pivots between -10º and +10º.

In some embodiments guide 10 may be limited to pivoting in only one direction. For example, guide 10 may be limited to pivoting between -10º and 0º, -20º and 0º, -30º and 0º, 0º and +10º, 0º and +20º, 0º and +30º, etc. In some such embodiments a portion of the bottom surface of protrusions 14 is curved (e.g. permits pivoting of guide 10) and a remaining portion of the bottom surface of protrusions 14 is flat (e.g. may prevent pivoting of guide 10). In some such embodiments the flat portion is substantially larger than the curved portion. In some embodiments the flat portion is at least three times wider than the curved portion. In some embodiments the flat portion is at least four times wider than the curved portion.

In some embodiments guide 10 may be pivoted more in one direction than an opposing direction. In some embodiments guide 10 may be pivoted between -5º and +20º, -25º and +15º, -10º and +30º, etc. In some such embodiments different portions of a bottom surface are shaped differently (e.g. have different radii of curvature, have different cross-sections) to facilitate more pivoting in one direction than an opposing direction.

In one example case (non-limiting) it is desirable to acquire ultrasound imaging data of one or more organs inside a thoracic cage of a patient. For example, it may be desirable to acquire a sweep of three-dimensional ultrasound imaging data of the patient's liver. In such case, guide 10 may be used to stabilize an ultrasound transducer within an intercostal space of the patient. A width of protrusions 14 may be dimensioned to approximately match average spacing between adjacent ribs of an average adult abdomen. Stabilizing the ultrasound transducer facilitates acquiring a sweep of three dimensional ultrasound data while the ultrasound transducer is pivoted about a single axis of rotation.

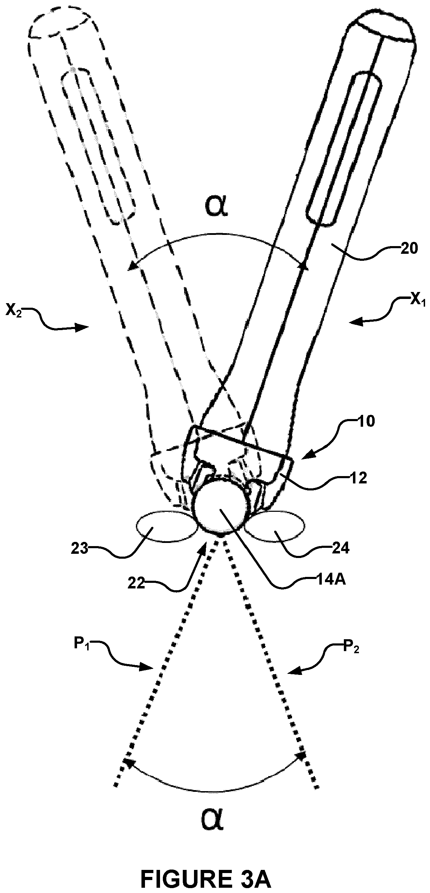

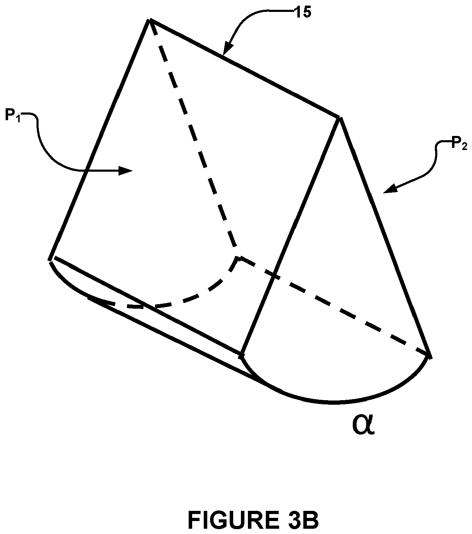

Figure 3 schematically illustrates an example use of guide 10 to stabilize an ultrasound transducer within an intercostal space of a patient. As shown in Figure 3, guide 10 is coupled to example ultrasound transducer 20 (may be a different transducer as described elsewhere herein). Protrusions 14 of guide 10 are aligned with and pressed into an intercostal space 22 between two adjacent ribs 23 and 24 of the patient. As described elsewhere herein, pressing protrusions 14 against the body of the patient secures guide 10 relative to the patient.

Once positioned proximate to intercostal space 22, transducer 20 (and guide 10) may be pivoted about axis 15. For example, transducer 20 (and guide 10) may be angularly pivoted by an angle α from a starting location X₁ to an ending location X₂.

Pivoting transducer 20 (and guide 10) from starting location X₁ to ending location X₂ acquires a sweep of three-dimensional ultrasound imaging data from an initial image plane P₁ to a final image plane P₂. In some embodiments ultrasound imaging data is collected continuously throughout the sweep. In such embodiments the data may be modelled as a wedge as shown in example Figure 3B. The point of the wedge is the axis of rotation (e.g. axis 15). In some embodiments ultrasound imaging data is collected periodically (e.g. at set angles, at set times, and/or the like). In some embodiments the number of ultrasound imaging planes captured throughout a sweep is reduced and/or limited to reduce processing time, data size and/or the like.

When performing an intercostal imaging scan of an organ inside a thoracic cage of the patient it is typically preferable for an axis of rotation and a face of an ultrasound transducer to be parallel to an axis extending through an intercostal space. Protrusions 14 may be positioned to be parallel to the axis extending through the intercostal space. Guide 10 may align a face of an ultrasound transducer to be parallel to the axis extending through the intercostal space. Additionally, or alternatively, protrusions 14 of guide 10 may center the transducer face within a region proximate to the intercostal space. Additionally, or alternatively, protrusions 14 may maintain the transducer in place while the transducer is pivoted.

Protrusions 14 may be made of the same or a different material as body 12. In some embodiments protrusions 14 are made of a softer (e.g. more pliable) material than the material used to make body 12. This may, for example, increase patient comfort. In some embodiments protrusions 14 are made of soft rubber. In some such embodiments body 12 is made of a harder plastic. Additionally, or alternatively, patient comfort may be increased by rounding corners of protrusions 14.

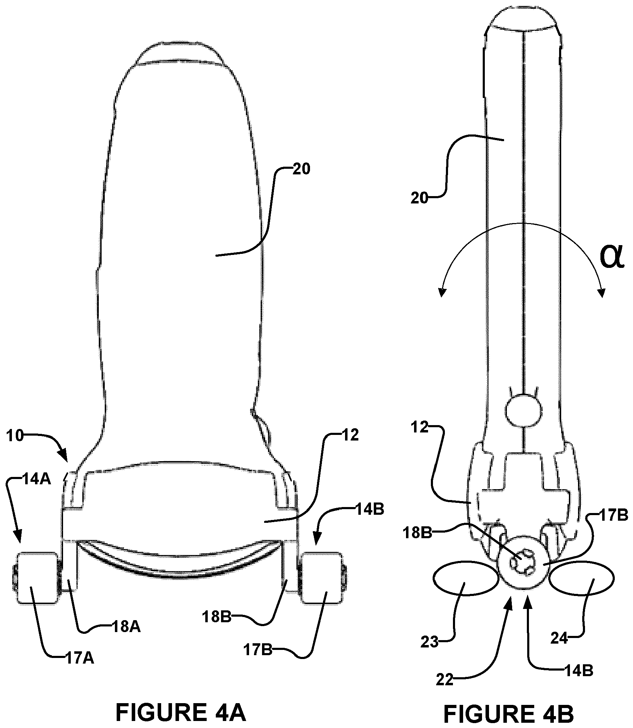

In some embodiments a protrusion 14 may comprise a fixed outer portion and a pivotable inner portion which may pivot relative to the fixed outer portion. For example, as shown in Figures 4A and 4B, a protrusion 14 may comprise an outer wheel 17 and an inner axle 18. Wheel 17 may be engaged with the body of a patient. Inner axle 18 may pivot relative to wheel 17 during a sweep of a coupled ultrasound transducer. Inner axle 18 may be pivotally coupled to outer wheel 17 using a bearing. The bearing may be positioned within an inner bore of wheel 17. In some embodiments inner axle 18 may freely pivot within an inner bore of wheel 17.

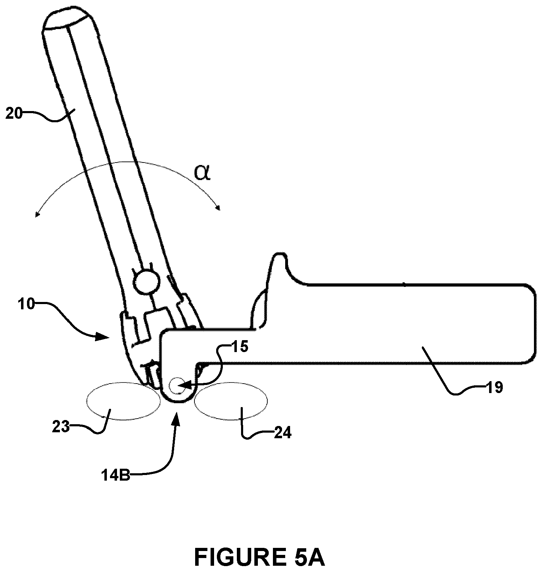

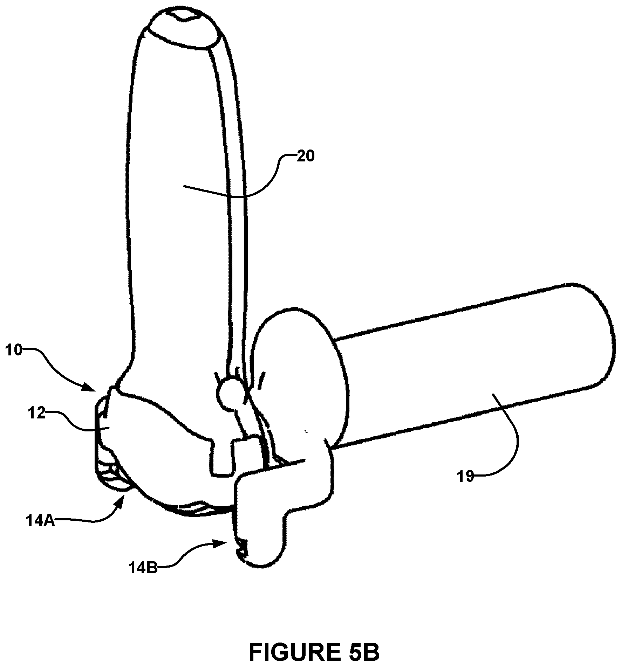

In some embodiments guide 10 comprises a handle 19 as shown in for example Figures 5A and 5B. In such embodiments a user may position guide 10 and a coupled ultrasound transducer (e.g. transducer 20) with one hand using handle 19 and use their other hand to pivot the transducer (e.g. about angle α).

In some embodiments protrusions 14 are integral with handle 19. In some such embodiments body 12 of guide 10 may comprise outwardly projecting axles which may be received within inner bores of protrusions 14 of handle 19. This may pivotally couple body 12 to handle 19. In some embodiments the inner bores of protrusions 14 comprise bearings for receiving the outwardly projecting axles. In some embodiments the outwardly projecting axles of body 12 are received within corresponding notches 14A, 14B of protrusions 14 (see e.g. Figure 5B).

It is convenient for the angle of handle 19 to be adjustable relative to body 12 of guide 10 but this is not mandatory. In some embodiments handle 19 is adjustable over a range of angles relative to body 12 of guide 10 (e.g. 0° to 45°, 0° to 60°, etc.). This may improve ease of use of guide 10 for a user when imaging different tissues of interest.

Protrusions 14A and 14B may be the same or different. In some cases, for example, one of protrusions 14A and 14B may have a smaller profile (e.g. a flat upper surface). This may for example assist with positioning guide 10 adjacent other medical equipment that may be present near the patient.

In some embodiments one or both of protrusions 14A and 14B are removably coupled to body 12 of guide 10. This may facilitate interchanging protrusions 14A and 14B. For example, protrusions 14A and 14B having different cross-sections may be coupled to body 12 of guide 10 for one patient and a different pair of protrusions 14A and 14B (e.g. a pair of protrusions 14 having the same cross-section) may be coupled to body 12 of guide 10 for a second patient.

A guide 10 may be coupled to an ultrasound transducer by a friction lock (e.g. cavity 13 is shaped to frictionally engage outer surfaces of the transducer). However this is not necessary. In some embodiments body 12 of guide 10 may be split into two or more portions (e.g. two opposing shells). The portions may be coupled together around the transducer (e.g. using fasteners, a strap, a clamp, a ratchet locking mechanism, etc.). In some embodiments cavity 13 comprises one or more grooves or recesses that engage corresponding projections of the transducer and/or one or more projections that engage one or more corresponding grooves or recesses in the transducer. In some embodiments guide 10 is fastened onto the ultrasound transducer (e.g. by an adhesive, fasteners, etc.).

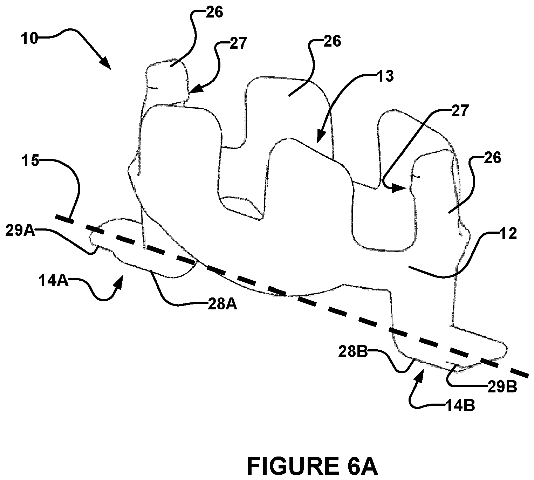

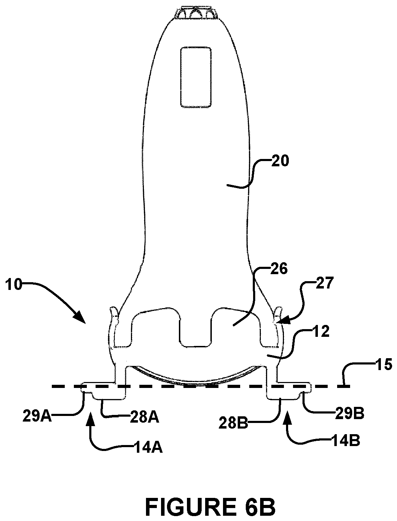

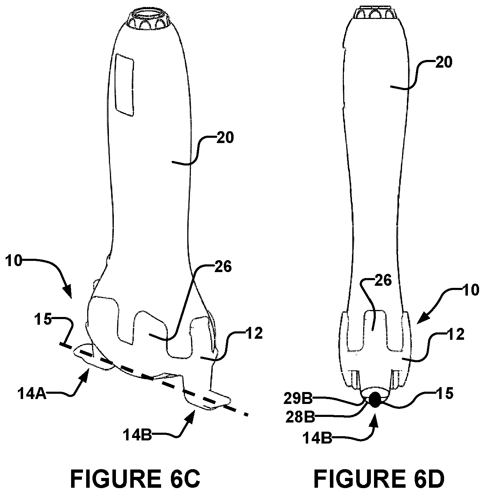

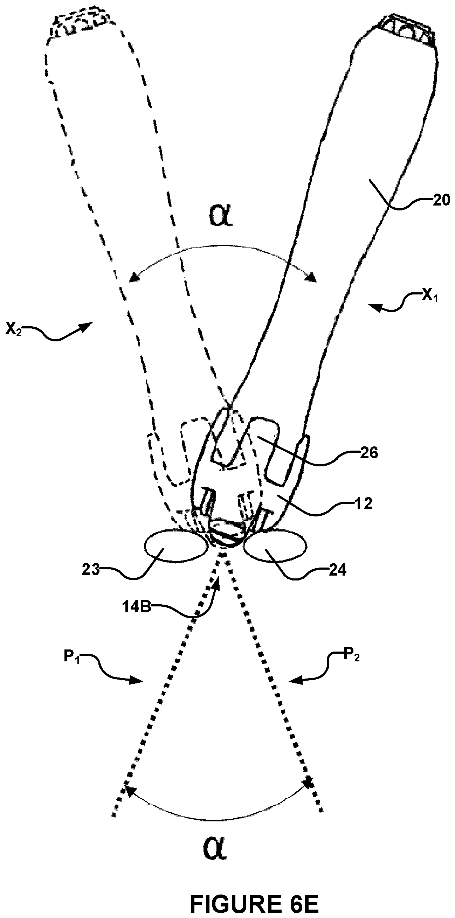

In some embodiments body 12 of guide 10 comprises fingers 26 (see e.g. Figure 6A). Fingers 26 may grip outer walls of an ultrasound transducer. In some embodiments cavity 13 defined by fingers 26 is slightly smaller than a corresponding cross-section of the ultrasound transducer. Fingers 26 may flex as the ultrasound transducer is introduced into cavity 13 (e.g. fingers 26 may be resilient fingers). This may, for example, provide a tight engagement between fingers 26 and the ultrasound transducer. In some embodiments one or more fingers 26 comprise features for engaging outer walls of the ultrasound transducer to lock guide 10 relative to the ultrasound transducer. For example, fingers 26 may comprise bumps 27 for engaging corresponding recesses in the ultrasound transducer. In some embodiments fingers 26 comprise recesses for engaging corresponding bumps on the ultrasound transducer.

Additionally, or alternatively, embodiments of guide 10 may comprise lower profile protrusions 14 as shown in Figures 6A to 6E. The lower profile protrusions may facilitate coupling additional components (e.g. a frame 30 described elsewhere herein) to guide 10. The lower profile protrusions may comprise a stepped bottom surface. For example, such protrusions may comprise a bottom surface comprising a lower portion 28 and an upper portion 29. The stepped bottom surface may assist with coupling a component to guide 10 (e.g. the stepped bottom surface may be received within a corresponding bore shaped to receive the stepped bottom surface).

In some cases, the stepped bottom surface extends the upper portion of protrusions 14. This may, for example, facilitate secure placement of an operator's fingers while allowing the shorter length lower portions to fit within a desired tissue region of a patient (e.g. within a tissue region in which protrusions 14 would not otherwise fit within if the lower and upper portions of protrusions 14 had the same width).

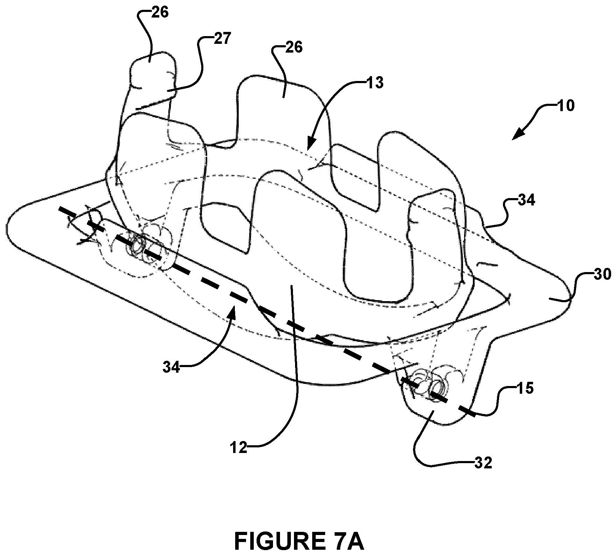

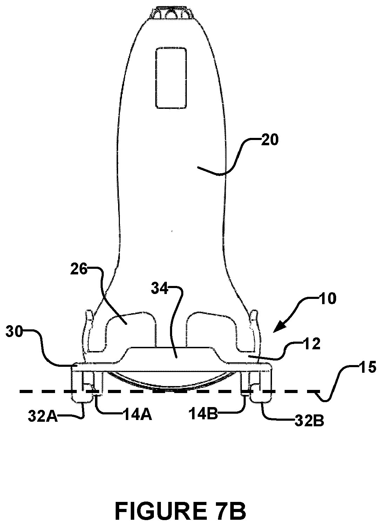

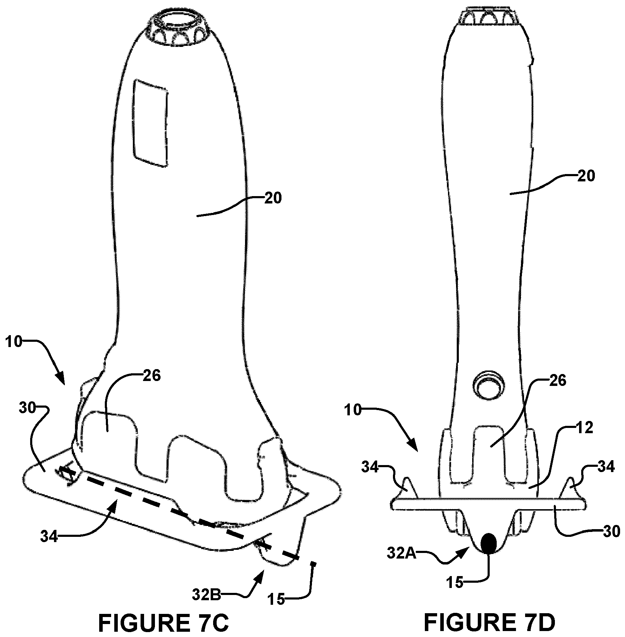

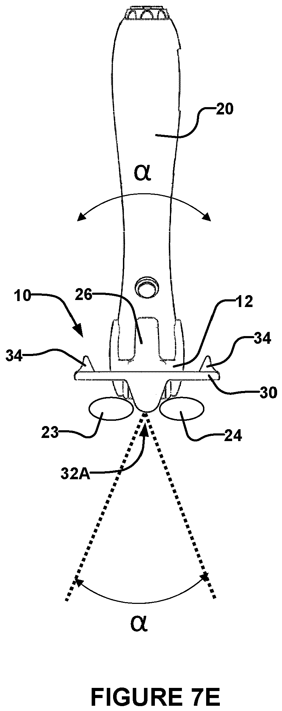

In some embodiments a frame 30 may be pivotally coupled to guide 10 (see e.g. Figures 7A to 7E). Frame 30 may increase a surface area of guide 10 that indents skin of the patient. This may, for example, assist with securing guide 10 relative to soft tissue regions of the patient which comprise no bone tissue against which guide 10 may be secured (e.g. a patient's belly) while guide 10 and the ultrasound transducer are pivoted. A bottom surface of frame 30 is preferably aligned with a face of the ultrasound transducer. Additionally, or alternatively, frame 30 may provide a larger surface area for an operator to hold the guide against a patient's skin during acquisition of the ultrasound imaging data. An operator may hold frame 30 at any point around the ultrasound transducer.

Frame 30 comprises protrusions 32. Protrusions 32 are typically similar to protrusions 14. Protrusions 32 may comprise inner bores configured to receive protrusions 14. Receiving protrusions 14 within the inner bores of protrusions 32 may pivotally couple frame 30 to guide 10. In some embodiments the inner bores of protrusions 32 comprise bearings. In some such embodiments protrusions 14 are received within bores of the bearings. Protrusions 32 typically project inwardly from opposing ends of frame 30 as shown in Figures 7A to 7E.

In some embodiments frame 30 comprises one or more ledges 34. Ledges 34 may limit how much guide 10 and the ultrasound transducer may pivot relative to frame 30. For example ledges 34 may prevent guide 10 and the ultrasound transducer from pivoting beyond a threshold angle of rotation (e.g. guide 10 abuts against a ledge 34 once guide 10 is pivoted by an amount equal to the threshold angle of rotation).

Additionally, or alternatively, one or more ledges may increase a surface area of frame 30 which may be held by an operator. For example, an operator's fingers of one hand may grip and push down against ledges 34 of frame 30 while using their other had to pivot the ultrasound transducer.

Although frame 30 has been shown as being rectangular, frame 30 may have any shape (e.g. circular, elliptical, hexagonal, octagonal, etc.).

Preferably guide 10 does not obstruct transmission of ultrasound energy between an ultrasound transducer coupled to guide 10 and a body of a patient being imaged. In some embodiments guide 10 covers no portion at all of an imaging face of a coupled ultrasound transducer.

A size of protrusions 14 may be varied to accommodate different patients, different imaging locations, etc.

In some embodiments guide 10 and the ultrasound transducer are integrated into a single component. For example, guide 10 may be integrated into an outer case of an ultrasound transducer.

In some embodiments the ultrasound transducer is pivoted manually (e.g. by an ultrasound operator). In some embodiments the ultrasound transducer is pivoted automatically. In some such embodiments the ultrasound transducer is pivoted by a robot or another mechanical system.

Guide 10 may be made of a suitable plastic, for example. In some embodiments guide 10 comprises injection-molded plastic or 3D printed plastic. In some embodiments guide 10 is made of metal, wood, resin, rubber and/or the like.

In some embodiments guide 10 is disposable (i.e. a "single-use" product). In some embodiments guide 10 may be disinfected and re-used. In some such embodiments guide 10 may be disinfected using a medical grade disinfectant.

In some embodiments guide 10 comprises a marker 16 (see e.g. Figure 1). Marker 16 may for example correspond to a marker of the ultrasound transducer. Marker 16 may for example indicate how a position along the guide correlates to a field of view of the ultrasound transducer. For example, marker 16 may indicate where a top of the field of view is, where a centre of the field of view is, etc. Additionally, or alternatively, if guide 10 is directional as discussed above (e.g. may be pivoted in only one direction, may be pivoted in one direction more than another direction, etc.), marker 16 may indicate the directionality of guide 10. Marker 16 may be an identifier that may be visually and/or tactilely located on body 12 of guide 10. Marker 16 may, for example, be a shaped bump or lump, a shaped recess, a notch, etc. In some embodiments marker 16 is included on at least two faces of guide 10.

In some embodiments body 12 of guide 10 is separated into an upper body portion and a lower body portion. The upper body portion may define cavity 13. The lower body portion may comprise protrusions 14. The upper body portion and the lower body portion may be coupled together. In some embodiments the upper body portion and the lower body portion are removably coupled together. In some embodiments the upper body portion and the lower body portion are coupled together using a hinge. In some embodiments the hinge is a snap hinge. The snap hinge may comprise first and second hinge elements that are detachably coupled together. In some embodiments the upper body portion and the lower body portion are coupled together using fasteners, a tongue and groove mechanism, a ball-and-socket joint, and/or the like.

In some embodiments a guide comprises a patient-contacting surface that is adapted to engage a surface of a patient and is coupled by a hinge to a body configured to be coupled to an ultrasound transducer. The hinge allows the body to pivot about an axis relative to the patient contacting surface. In use, the patient contacting surface may be placed against the patient at a reference location such that a transducer array of an ultrasound transducer coupled to the body is against the patient. The reference location may, for example, be between two ribs of the patient. The body may be located such that images are acquired by the ultrasound transducer through an intercostal space of the patient. The images may, for example, include all or portions of the patient's liver.

The hinge permits the transducer to be pivoted about a pivot axis that is generally parallel to a surface of the patient to obtain ultrasound images that may be combined to yield a 3D image as described elsewhere herein.

The pivot axis may be located to lie in an imaging plane of the ultrasound transducer. The pivot axis may extend parallel to a longitudinal axis of an ultrasound transducer array. As the ultrasound transducer is pivoted to direct an imaging plane in different directions the pivot axis may have a common location in all of the images. In some embodiments the hinge comprises first and second separable hinge parts which can be separated to allow the patient contacting surface to be separated from the body. The patient-contacting surface may, for example, be provided by a protrusion as described in various embodiments elsewhere herein or by an alternative surface such as a surface of a pad or plate.

Three dimensional reconstruction of acquired ultrasound imaging data has been described relative to a fixed axis. However, this is not necessary in all cases. In some embodiments three dimensional reconstruction of acquired ultrasound imaging data is performed relative to a fixed point relative to a face of the ultrasound transducer (e.g. a mid-point between protrusions 14A and 14B). The fixed point may be common to a plurality of ultrasound images acquired by the transducer. The known location of the fixed point in the images may be used to align the images relative to one another so that the images may be processed to provide a 3D data structure.

Although guide 10 has been illustrated as comprising two protrusions 14 herein, this is not necessary in all cases. In some embodiments guide 10 has a single protrusion 14. Engaging the single protrusion 14 with skin of a patient may provide a fixed point about which an ultrasound transducer may be pivoted.

In some cases a face of an ultrasound transducer may provide one protrusion and a guide 10 coupled to the ultrasound transducer which has a single protrusion 14 provides the second protrusion. This may be particularly advantageously for small sized probes (e.g. having a face with a width in the range of about 0.5 to 2 cm).

In any embodiment described herein a ultrasound transducer array of an ultrasound transducer may have a suitable arrangement of transducer elements. For example, a transducer array may comprise any of:
- A 1D array of transducer elements such as a straight linear array of transducer elements or a curved linear array of transducer elements;
- A 2D array of transducer elements.

In some embodiments an ultrasound transducer is capable of acquiring 3D ultrasound image data without being pivoted or without being moved. For example, a guide as described herein may be used to support an ultrasound transducer that includes a 2D transducer array controlled by an ultrasound machine to acquire 3D ultrasound image data. One or more protrusions and/or patient contacting surfaces of the guide may be used to align the ultrasound transducer array over an intercostal space of the patient to allow 3D ultrasound imaging through the intercostal space. The one or more protrusions and/or patient contacting surfaces may, for example, be engaged between the patient's ribs while the 3D ultrasound imaging is being performed.

Although guide 10 has been explained in the context of imaging human patients, guide 10 may also be used to image animal patients (domestic or wild animals).

### Interpretation of Terms

Unless the context clearly requires otherwise, throughout the description and the claims:
- "ultrasound image" means any image obtained using ultrasound including, for example, B-mode ultrasound images, Doppler ultrasound images, ultrasound elastography images etc.
- "comprise", "comprising", and the like are to be construed in an inclusive sense, as opposed to an exclusive or exhaustive sense; that is to say, in the sense of "including, but not limited to";
- "connected", "coupled", or any variant thereof, means any connection or coupling, either direct or indirect, between two or more elements; the coupling or connection between the elements can be physical, logical, or a combination thereof;
- "herein", "above", "below", and words of similar import, when used to describe this specification, shall refer to this specification as a whole, and not to any particular portions of this specification;
- "or", in reference to a list of two or more items, covers all of the following interpretations of the word: any of the items in the list, all of the items in the list, and any combination of the items in the list;
- the singular forms "a", "an", and "the" also include the meaning of any appropriate plural forms.

Words that indicate directions such as "vertical", "transverse", "horizontal", "upward", "downward", "forward", "backward", "inward", "outward", "left", "right", "front", "back", "top", "bottom", "below", "above", "under", and the like, used in this description and any accompanying claims (where present), depend on the specific orientation of the apparatus described and illustrated. The subject matter described herein may assume various alternative orientations. Accordingly, these directional terms are not strictly defined and should not be interpreted narrowly.

Specific examples of systems, methods and apparatus have been described herein for purposes of illustration. These are only examples. The technology provided herein can be applied to systems other than the example systems described above. Many alterations, modifications, additions, omissions, and permutations are possible within the practice of this invention. This invention includes variations on described embodiments that would be apparent to the skilled addressee, including variations obtained by: replacing features, elements and/or acts with equivalent features, elements and/or acts; mixing and matching of features, elements and/or acts from different embodiments; combining features, elements and/or acts from embodiments as described herein with features, elements and/or acts of other technology; and/or omitting combining features, elements and/or acts from described embodiments.

Various features are described herein as being present in "some embodiments". Such features are not mandatory and may not be present in all embodiments. Embodiments of the invention may include zero, any one or any combination of two or more of such features. This is limited only to the extent that certain ones of such features are incompatible with other ones of such features in the sense that it would be impossible for a person of ordinary skill in the art to construct a practical embodiment that combines such incompatible features. Consequently, the description that "some embodiments" possess feature A and "some embodiments" possess feature B should be interpreted as an express indication that the inventors also contemplate embodiments which combine features A and B (unless the description states otherwise or features A and B are fundamentally incompatible).

## Claims

1. A guide for guiding pivotal movement of an ultrasound transducer relative to a volume to be imaged, the guide comprising:
a body configured to couple to an end of the ultrasound transducer that includes a transducer array and first and second outwardly extending protrusions respectively located at first and second opposing ends of the body such that:
the first and second protrusions are located at first and second ends of the transducer array; and
a pivot axis is defined by the first and second protrusions such that when the body is coupled to the ultrasound transducer the ultrasound transducer is pivotable relative to the pivot axis;
each of the first and second protrusions comprising a patient-contacting surface that projects forwardly relative to the pivot axis, the first and second protrusions operable to indent skin of a patient adjacent to a region of the patient to be imaged by the ultrasound transducer when the ultrasound transducer is pressed against the skin of the patient.

2. The guide according to claim 1 wherein the pivot axis is aligned with a front surface of the transducer array.

3. The guide according to claim 1 or 2 wherein the pivot axis is in an imaging plane of the transducer array.

4. The guide according to any of claims 1 to 3 wherein the body is removably attachable and detachable from the ultrasound transducer.

5. The guide according to claim 4 wherein the body defines a cavity shaped to receive the end of the ultrasound transducer that includes the transducer array, the cavity having an opening aligned with the transducer array when the transducer is inserted into the cavity and wherein the cavity is formed to provide a positive stop when the front face of the transducer array has a desired alignment with the pivot axis.

6. The guide according to any of claim 5 wherein the body comprises a plurality of resilient fingers spaced around an opening of the cavity, the resilient fingers dimensioned to flex when the end of the transducer is inserted into the cavity.

7. The guide according to any of claims 1 to 6 wherein a patient-contacting surface of each of the protrusions has a radius of curvature substantially equal to a distance by which the patient-contacting surface projects forwardly relative to the front surface of the transducer array and wherein preferably the radius of curvature is in the range of about 0.5 cm to 1 cm.

8. The guide according to any of claims 1 to 7 wherein the patient-contacting surfaces of the first and second protrusions each comprises a cylindrical configuration.

9. The guide according to any of claims 1 to 8 wherein the first and second protrusions are each mounted to pivot relative to the body and wherein preferably each of the first and second protrusions comprises a roller mounted to rotate about the pivot axis.

10. The guide according to any of claims 1 to 9 comprising a handle pivotally mounted to the body;
wherein preferably
the handle extends generally perpendicularly to the pivot axis;
the handle is pivotal relative to the body about the pivot axis;
the handle comprises first and second arms that are respectively pivotally mounted to the body adjacent to the first and second ends of the body; and/or
the protrusions are provided by end portions of the first and second arms.

11. The guide according to claim 10 wherein the handle comprises a frame and the frame includes one or more stops positioned to limit angular travel of the body relative to the frame.

12. The guide according to any of claims 1 to 11 wherein the first and second protrusions are detachably mounted to the body.

13. The guide according to any of claims 1 to 12 comprising a mechanism for adjusting a distance by which at least one of the first second protrusions projects forwardly from the pivot axis.

14. The guide according to any of claims 1 to 13 wherein the first and second protrusions have dimensions parallel to the pivot axis in the range of ½ cm to 2 cm.

## Patentansprüche

1. Eine Führung zum Führen einer Schwenkbewegung eines Ultraschallwandlers relativ zu einem abzubildenden Volumen, wobei die Führung Folgendes umfasst:
ein Gehäuse, das eingerichtet ist, an ein Ende des Ultraschallwandlers zu koppeln, das eine Wandleranordnung umfasst, und erste und zweite nach außen vorstehende Teile, die jeweils an ersten und zweiten, gegenüberliegenden Enden des Gehäuses angebracht sind, sodass:
die ersten und zweiten vorstehenden Teile an ersten und zweiten Enden der Wandleranordnung angeordnet sind; und
eine Schwenkachse von den ersten und zweiten vorstehenden Teilen definiert wird, sodass, wenn das Gehäuse an den Ultraschallwandler gekoppelt ist, der Ultraschallwandler relativ zu der Schwenkachse schwenkbar ist;
wobei jedes der ersten und zweiten vorstehenden Teile eine patientenkontaktierende Oberfläche umfasst, die relativ zu der Schwenkachse nach vorne ragt, wobei die ersten und zweiten vorstehenden Teile geeignet sind, die Haut eines Patienten in der Nähe einer von dem Ultraschallwandler abzubildenden Region des Patienten einzudrücken, wenn der Ultraschallwandler gegen die Haut des Patienten gedrückt wird.

2. Die Führung nach Anspruch 1, wobei die Schwenkachse an einer vorderen Oberfläche der Wandleranordnung ausgerichtet ist.

3. Die Führung nach Anspruch 1 oder 2, wobei die Schwenkachse in einer Bildaufnahmeebene der Wandleranordnung liegt.

4. Die Führung nach einem der Ansprüche 1 bis 3, wobei das Gehäuse abnehmbar zu befestigen und von dem Ultraschallwandler zu lösen ist.

5. Die Führung nach Anspruch 4, wobei das Gehäuse einen Hohlraum ausbildet, der geformt ist, das Ende des Ultraschallwandlers, das die Wandleranordnung umfasst, aufzunehmen, wobei der Hohlraum eine Öffnung hat, die an der Wandleranordnung ausgerichtet ist, wenn der Wandler in den Hohlraum eingeführt ist, und wobei der Hohlraum geformt ist, einen positiven Anschlag zu bilden, wenn die vordere Fläche der Wandleranordnung eine gewünschte Ausrichtung an der Schwenkachse hat.

6. Die Führung nach Anspruch 5, wobei das Gehäuse eine Vielzahl an nachgiebigen Fingern umfasst, die um eine Öffnung des Hohlraums herum angeordnet sind, wobei die nachgiebigen Finger dimensioniert sind, sich zu biegen, wenn das Ende des Wandlers in den Hohlraum eingeführt wird.

7. Die Führung nach einem der Ansprüche 1 bis 6, wobei eine patientenkontaktierende Oberfläche jedes der vorstehenden Teile einen Krümmungsradius hat, der im Wesentlichen gleich einer Distanz ist, über welche die patientenkontaktierende Oberfläche relativ zu der vorderen Oberfläche der Wandleranordnung nach vorne ragt und wobei der Krümmungsradius bevorzugt im Bereich von etwa 0,5 cm bis 1 cm liegt.

8. Die Führung nach einem der Ansprüche 1 bis 7, wobei die patientenkontaktierenden Oberflächen der ersten und zweiten vorstehenden Teile jeweils eine zylindrische Ausgestaltung aufweisen.

9. Die Führung nach einem der Ansprüche 1 bis 8, wobei die ersten und zweiten vorstehenden Teile jeweils relativ zu dem Gehäuse schwenkbar befestigt sind und wobei bevorzugt jedes der ersten und zweiten vorstehenden Teile eine Rolle umfasst, die geeignet befestigt ist, um um die Schwenkachse herum zu rotieren.

10. Die Führung nach einem der Ansprüche 1 bis 9, wobei die Führung einen schwenkbar an dem Gehäuse befestigten Griff umfasst;
wobei bevorzugt
der Griff im Allgemeinen senkrecht zu der Schwenkachse angebracht ist; der Griff relativ zu dem Gehäuse um die Schwenkachse herum schwenkbar ist;
der Griff erste und zweite Arme umfasst, die jeweils nahe den ersten und zweiten Enden des Gehäuses schwenkbar an dem Gehäuse befestigt sind; und/oder
die vorstehenden Teile von den Endabschnitten der ersten und zweiten Arme gebildet werden.

11. Die Führung nach Anspruch 10, wobei der Griff einen Rahmen umfasst und der Rahmen einen oder mehrere Anschläge umfasst, die geeignet positioniert sind, um eine Winkelbewegung des Gehäuses relativ zu dem Rahmen einzuschränken.

12. Die Führung nach einem der Ansprüche 1 bis 11, wobei die ersten und zweiten vorstehenden Teile abnehmbar an dem Gehäuse befestigt sind.

13. Die Führung nach einem der Ansprüche 1 bis 12, wobei die Führung einen Mechanismus zum Anpassen einer Distanz, über die mindestens eines der ersten und zweiten vorstehenden Teile über die Schwenkachse hinaus nach vorne ragt, umfasst.

14. Die Führung nach einem der Ansprüche 1 bis 13, wobei die ersten und zweiten vorstehenden Teile zur Schwenkachse parallele Dimensionen im Bereich von 0,5 cm bis 2 cm haben.

## Revendications

1. Guide pour guider un mouvement pivotant d'un transducteur ultrasonore par rapport à un volume à imager, le guide comprenant :
un corps configuré pour se coupler à une extrémité du transducteur ultrasonore qui comporte un réseau de transducteur et des première et deuxième saillies s'étendant vers l'extérieur, situées respectivement à des première et deuxième extrémités opposées du corps, de sorte que :
les première et deuxième saillies soient situées à des première et deuxième extrémités du réseau de transducteur ; et
un axe de pivotement soit défini par les première et deuxième saillies, de sorte que lorsque le corps est couplé au transducteur ultrasonore, le transducteur ultrasonore puisse pivoter par rapport à l'axe de pivotement ;
chacune des première et deuxième saillies comprenant une surface en contact avec le patient qui fait saillie vers l'avant par rapport à l'axe de pivotement, les première et deuxième saillies pouvant être utilisées pour mettre en retrait la peau d'un patient de manière adjacente à une région du corps du patient à imager par le transducteur ultrasonore lorsque le transducteur ultrasonore est pressé contre la peau du patient.

2. Guide selon la revendication 1, dans lequel l'axe de pivotement est aligné avec une surface avant du réseau de transducteur.

3. Guide selon la revendication 1 ou 2, dans lequel l'axe de pivotement se trouve dans un plan d'imagerie du réseau de transducteur.

4. Guide selon l'une des revendications 1 à 3, dans lequel le corps peut être fixé de manière amovible et détaché du transducteur ultrasonore.

5. Guide selon la revendication 4, dans lequel le corps définit une cavité formée pour recevoir l'extrémité du transducteur ultrasonore qui comporte le réseau de transducteur, la cavité ayant une ouverture alignée avec le réseau de transducteur lorsque le transducteur est inséré dans la cavité, et dans lequel la cavité est formée pour fournir une butée positive lorsque la face avant du réseau de transducteur présente un alignement souhaité avec l'axe de pivotement.

6. Guide selon la revendication 5, dans lequel le corps comprend une pluralité de doigts élastiques espacés autour d'une ouverture de la cavité, les doigts élastiques étant dimensionnés pour fléchir lorsque l'extrémité du transducteur est insérée dans la cavité.

7. Guide selon l'une des revendications 1 à 6, dans lequel une surface en contact avec le patient de chacune des saillies a un rayon de courbure sensiblement égal à une distance à laquelle la surface en contact avec le patient fait saille vers l'avant par rapport à la surface avant du réseau de transducteur et dans lequel, de préférence, le rayon de courbure est dans la plage d'environ 0,5 cm à 1 cm.

8. Guide selon l'une des revendications 1 à 7, dans lequel les surfaces en contact avec le patient des première et deuxième saillies comprennent chacune une configuration cylindrique.

9. Guide selon l'une des revendications 1 à 8, dans lequel les première et deuxième saillies sont chacune montées pour pivoter par rapport au corps et dans lequel, de préférence, chacune des première et deuxième saillies comprend un rouleau monté pour tourner autour de l'axe de pivotement.

10. Guide selon l'une des revendications 1 à 9 comprenant une poignée montée pivotante sur le corps ;
dans lequel, de préférence
la poignée s'étend globalement perpendiculairement à l'axe de pivotement ;
la poignée est pivotante par rapport au corps autour de l'axe de pivotement ;
la poignée comprend des premier et deuxième bras qui sont respectivement montés de manière pivotante sur le corps de manière adjacente aux première et deuxième extrémités du corps ; et/ou
les saillies sont fournies par des parties d'extrémité des premier et deuxième bras.

11. Guide selon la revendication 10, dans lequel la poignée comprend un cadre et le cadre comporte une ou plusieurs butées positionnées pour limiter un déplacement angulaire du corps par rapport au cadre.

12. Guide selon l'une des revendications 1 à 11, dans lequel les première et deuxième saillies sont montées de manière amovible sur le corps.

13. Guide selon l'une des revendications 1 à 12, comprenant un mécanisme pour régler une distance à laquelle au moins l'une des première et deuxième saillies fait saillie vers l'avant depuis l'axe de pivotement.

14. Guide selon l'une des revendications 1 à 13, dans lequel les première et deuxième saillies ont des dimensions parallèles à l'axe de pivotement dans la plage de ½ cm à 2 cm.
